# EUROPEAN PATENT SPECIFICATION

(11) **EP 3 484 843 B1**
(45) Date of publication and mention of the grant of the patent: **26.02.2020**
(21) Application number: 17751134.2
(22) Date of filing: 10.07.2017
(51) Int. Cl.: C07C 37/20, C07C 37/84, C07C 39/16

(54) **MANUFACTURE OF BISPHENOL A**
HERSTELLUNG VON BISPHENOL A
PRODUCTION DE BISPHÉNOL A

(30) Priority: 12.07.2016 EP 16179051
(43) Date of publication of application: 22.05.2019
(73) Proprietor: SABIC Global Technologies B.V., 4612 PX Bergen op Zoom (NL)
(72) Inventor: EIJSBOUTS, Paulus Johannes Maria, 4612 PX Bergen op Zoom (NL); HEININK, Rene, 4612 PX Bergen op Zoom (NL)
(74) Representative: Balder IP Law, S.L.
(86) International application number: PCT/IB2017/054153
(87) International publication number: WO 2018/011700

(56) References cited:
- EP-A1- 0 671 377
- EP-A1- 1 728 777
- FR-A- 1 287 250
- US-A- 3 326 986
- US-A- 5 648 561
- US-B1- 8 680 227

## Description

The invention is directed to a method for the manufacture of bisphenol, to the use of acid in a method for crystallisation of bisphenol A/phenol adduct, and to bisphenol A/phenol adduct crystals.

Bisphenol A 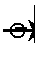 (2,2-bis(4-hydroxyphenyl)propane, also known as p,p-BPA) is predominantly used as an intermediate for the production of other products. The main uses of bisphenol A are binding, plasticising, and hardening functions in plastic products, paints/lacquers, binding materials and filler materials. The primary use for bisphenol A is in the production of polycarbonate resins, epoxy resins, unsaturated polyester, polysulphone, polyetherimide and polyarylate resins.

Bisphenol A is commercially prepared by condensing two moles of phenol with a mole of acetone in the presence of an acid catalyst as shown in equation (1) below.

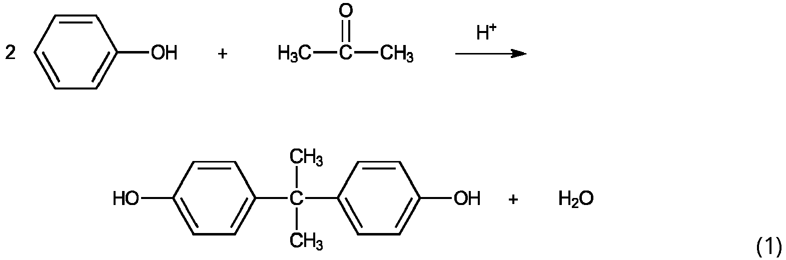

The phenol is present in the reaction in a molar excess of the stoichiometric requirement. During the condensation, a number of by-products such as isomeric forms of the bisphenol A are formed which are considered contami nants of the desired product The main contami nants present are the 2,4 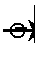 bisphenol isomer (2-(2-hydroxyphenyl)-2-(4-hydroxyphenyl)propane also known as o,p-bisphenol A), 2,4-bis(a,a-dimethyl-4-hydroxybenzyl) phenol also known as BPX-1, 2,4-bis(1-(4-hydroxyphenyl)-1-phenol also known as BPX-2, 4-(4 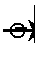 hydroxyphenyl)-2,2,4-trimethylchroman also known as codimer or Dianin's compound, and 2-(4 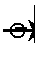 hydroxyphenyl)-2,4,4-trimethylchroman also known as isomeric codimer or an isomer of Dianin's compound. For the avoidance of doubt the formulas below show the chemical structure of some of these most important contaminants:

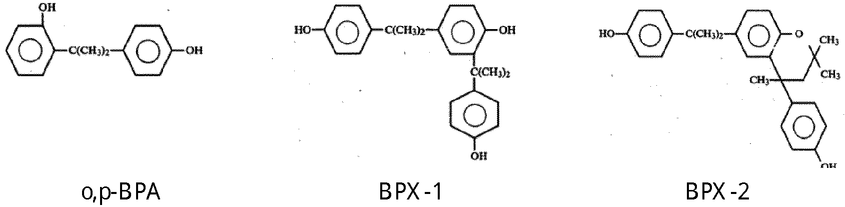

These contami nants are carried in the product stream from the condensation reaction zone, with water, trace quantities of acidic materials derived from the catalyst, unreacted phenol and unreacted acetone. When bisphenol A is used for preparing polycarbonate polymer, it is however required that the bisphenol A is very pure, because these contami nants have the tendency to form colour bodies under the alkaline reacti on conditions used in polycarbonate production. In particular, for so-called melt polycarbonate, which is polycarbonate made in a process where bisphenol A and a second reactant (diphenol carbonate) are reacted in melt phase, it is important the bisphenol A used is very pure. Currently, the purification of the desired product bisphenol A is a costly and multi-step procedure.

Commercial processes for purifying bisphenol A out of the reaction mixture of p,p-BPA with its various isomers and by-products use crystallisation as key process step. The mixture can be untreated reactor effluent, in which case excess phenol is present that acts as solvent, or the reaction effluent may be treated to remove excess water, acetone and phenol, in which case the mixture is concentrated in phenol or even phenol free. In the crystallisation step, the reaction mixture is cooled to form a slurry containing a crystalline bisphenol A/phenol adduct. In case of a concentrated or phenol free reaction mixture, the mixture can be dissolved (or diluted) in a solvent at elevated temperature (solvents can be various aromatic, chlorinated al kanes, or simple al kanes) before cooling down the solution. Due to the cooling process, possi bly combined by solvent removal to increase the concentration, crystals are created that have a high concentration of the desired isomer, being the p,p-BPA isomer. The slurry is then filtered to remove the excess liquid and washed with clean solvent to obtain a cake (crystal mass) that is as free as possible of the liquid phase.

These commercial processes all have the disadvantage that the crystal is still contaminated to a large extent by two specific isomer species: mainly o,p-BPA and BPX-2. In order to obtain high purity p,p-BPA of more than 99.90 % purity, commercial processes use a two-step crystallisation approach in which the crystal mass is redissolved in a solvent and recrystallized, or in which the crystal mass is molten and subjected to a melt crystallisation process (e.g. WO-A-02/40435, E P-A-0 718 268, E P-A-1 367 043, and E P-A-1 728 777). This requires significant investment and operational costs.

Another solution is proposed in EP-A-0 671 377 by going through an adduct crystallisation process with two different temperature level s at extremely long residence times of at least 9 hours in each step (for this high purity). Such long residence times require very big vessels and are economically not attractive for bulk chemical production.

FR-A-1 287 250 describes a process for preparing pure 4,4 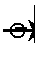 dihydroxydiarylalkanes wherein hydrochloric acid is used as acid catalyst. Excess aqueous hydrochloric acid solution is added to a mixture of phenol and acetone, to form bisphenol A/phenol adduct crystals. A significant amount of acid is present during subsequent crystallisation.

US-A-5 648 561 describes a process for the production of high purity bisphenol A. In the process phenol and acetone are reacted in the presence of catalyst to form bisphenol A/phenol adduct crystals, wherein the reaction is performed in a special reactor column. Phenol may be removed from the bisphenol A/phenol adduct crystals by using a vacuum dephenoliser and an inert gaseous flow dephenoliser. Subsequently, the obtained bisphenol A crystals may be recrystallized for further purification.

There remains a need in the art for the production of highly pure bisphenol A in a relatively simple and cost-effective manner.

Objective of the invention is to overcome at least part of the disadvantages faced in the prior art.

The inventors found that this objective can, at least in part, be met by performing the crystallisation step in the presence of a specific compound.

A ccordi ngly, in a first aspect the invention is directed to a method for the manufacture of bisphenol A comprising
a) reacting phenol and acetone in the presence of an acidic catalyst to form a reaction product comprising bisphenol A and
b) crystallising a bisphenol/phenol adduct from said reaction product, wherein said crystallisation is carried out in the presence of an acid, wherein total amount of acid present during the crystallisation step is 5 % or less by total weight of the reaction product

The inventors surprisingly found that the presence of an acid in the crystallisation step can effectively suppress co-crystallisation of undesirable by-products, thereby directly resulting in a very high purity crystal in a simple, economical crystallisation process.

The first step of reacting phenol and acetone in the presence of an acidic catalyst to form a reaction product comprising bisphenol A is commonly known in the art.

The molar ratio of phenol to acetone is usually in the range of 3-30 moles of phenol per mole of acetone, and preferably 5-15 moles of phenol per mole of acetone. If the molar ratio is smaller than 3 moles of phenol per mole of acetone, then the reaction speed is likely to be too slow. If it is larger than 30 moles of phenol per mole of acetone then the reaction product (e.g., amount of *p*,*p-*bisphenol A) becomes too dilute to have commercial significance. In other words, the amount of desired reaction product is too low, making it difficult to efficiently isolate it from the product mixture.

The reacti on temperature can be 40-150 °C, preferably 60-110 °C, more preferably 50-100 °C. If the reaction temperature is lower than 40 °C, not only the reaction speed is slow but also the reaction solution has a very high viscosity and may solidify. On the other hand, if the reaction temperature exceeds 150 °C, it becomes difficult to control the reaction, and a selectivity of bisphenol A (p,p-BPA) is lowered. In addition, the catalyst may be decomposed or deteriorated.

The acidic catalyst can be a homogenous acidic catalyst or a solid acidic catalyst. In view of low corrosiveness of devices and easiness in separating the catalyst from the reaction mixture, solid acidic catalysts are preferred.

It is preferred that the reaction of step a) is performed in the presence of a limited amount of water. High levels of water (e.g., greater than 10%) can impact the crystallization process by suppressing the crystallization temperature, and can impact the crystal shape and distribution. Accordingly, the amount of water present during the reaction of step a) may be 10 % or less by total weight of the reaction mixture, preferably 5 % or less, even more preferably 3 % or less. The reaction of step a) may also be performed in the absence of water.

When a homogenous acidic catalyst is used, hydrochloric acid or sulphuric acid are generally used. Sulphuric acid is preferably used since it can be easily separated. When a solid acidic catalyst is used, a sulphonic acid-type ion-exchange resin is generally used. Examples thereof include sulphonated styrene-divinylbenzene copolymers, sulphonated cross-linked styrene polymers, phenol formal dehyde-sulphonic acid resins, and benzene formaldehyde-sulphonic acid resins. These catalysts may be used individually or in combination.

The reaction of step a) may be performed batch-wise or continuously. Preferably, the reaction is performed in a fixed bed continuous reactor in which phenol and acetone are continuously fed into a reactor filled with an acid-type ion-exchange resin to react them. The reactor may be a single reactor, or may be two or more reactors that are connected in series.

Optionally, the reaction mixture of step a) is subjected to a step for removing unreacted acetone, and water, e.g. by distillation. Such optional distillation may be performed under reduced pressure using a distillation column. In general such distillation is carried out at a pressure of 6.5-80 kPa and at a temperature of 70-180 °C. Unreacted phenol then boils by azeotropy, and part thereof is removed.

Optionally, the bisphenol A may be concentrated by further removal of phenol. Such further distillation may typically be performed at 100-170 **°C** and a pressure of 5-70 kPa.

The reaction product obtained in step a) usually includes, in addition to bisphenol A, unreacted acetone, unreacted phenol, water produced during the reaction and other by-products, which have a combined total weight of 100 wt%. The reaction product typically comprises
- at least 10 % by total weight of the reaction product of bisphenol A, preferably 15-40 %,
- 60-85 % by total weight of the reaction product of phenol, preferably 65-80 %,
- 0-5 % by total weight of the reaction product of water, preferably 0-3 %,
- 0-3 % by total weight of the reacti on product of acetone, preferably 0-0.5 %, and/or
- 0-20 % by total weight of the reaction product of by-products, preferably 2-16 %.

When the concentration of bisphenol A is less than 10 % by total weight of the reaction product, the recovery rate of adduct crystals becomes low. When the concentration of bisphenol A is more than 50 % by total weight of the reaction product, the viscosity of the slurry becomes high, so that the transportation of the slurry becomes difficult.

The acid can simply be added to the reaction product obtained in step a). Suitably, the mixture is stirred upon addition of the acid.

The acid used in the crystallisation step b) can comprise an inorganic acid, an organic acid, or a mixture thereof. In an embodiment the acid comprises or is an inorganic acid. In another embodiment the acid comprises or is an organic acid.

Inorganic acids can be used in the crystallisation step in amounts of 1 % or less by total weight of the reaction product, preferably 0.8 % or less, such as 0.5-0.01 %, or 0.3-0.02 %. The amount of acid to be added can thus be determined based on the mass of the product directly obtained from the reaction, regardless the possible removal or addition of components.

The inorganic acid is preferably a strong acid, which is defined as an acid which completely dissociates in an aqueous solution.

The inorganic acid is preferably selected from the group consisting of hydrochloric acid, hydrobromic acid, hydrofluoric acid, perchloric acid, boric acid, sulphuric acid, phosphoric acid, nitric acid and any mixtures thereof. More preferably, the inorganic acid is selected from the group consisting of hydrochloric acid, phosphoric acid, nitric acid and any mixtures thereof.

Organic acids can be used in the crystallisation step in amounts of 5 % or less by total weight of the reaction product, preferably as 4.5 % or less, such as 0.2-4 %, or 0.4-3.5 %. In other words, the organic acid can be used in an amount from more than zero up to 5%, preferably from more than zero up to 4.5%, based on the total weight of the reaction product.

The organic acid is preferably selected from the group consisting of organic acids having a pKₐ (relative to water) of 3.3 or less, more preferably a pKₐ of 3 or less. In case the acid has more than one dissociation constant, then the pKₐ as mentioned herein refers to the first dissociation constant Some examples of organic acids having a pKₐ of 3.3 or less include glyoxylic acid, oxalic acid, trichloroacetic acid, trifluoroacetic acid, methane sulphonic acid, pyruvic acid, malonic acid, lactic acid, fumaric acid, maleic acid, tartaric acid, aspartic acid, mesaconic acid, glutamic acid, picric acid, picolinic acid, benzenesulphonic acid, toluene sulphonic acid, aconitic acid, citric acid, lutidinic acid, and quinolinic acid.

Suitable acids may also include Lewis acids, such as BF₃, SnCl₄, AlCl₃, Al(CH₃)₂Cl, and ZnCl₂. Lewis acids are less preferred than Br 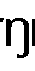 nsted acids, because they may give rise to side reactions with bisphenol A. Hence, the acid is preferably a Br 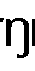 nsted acid.

The crystallisation may be carried out in the presence of an individual acid, or in the presence of two or more acids, such as an inorganic acid and an organic acid as described above. For example, the crystallisation may be carried out in the presence of a strong inorganic acid and a weak organic acid.

The total amount of acid present during the crystallisation step is 5 % or less by total weight of the reaction product, preferably 4 % or less, more preferably 3 % or less. In other words, the amount of acid present during the crystallisation step is more than zero by total weight of the reaction product, preferably more than 0.005 %, more preferably more than 0.01%; e.g., more than zero up to 5 % by total weight of the reaction product, preferably 0.01 to 4%, more preferably 0.02 to 3 %. Typically, the acid is added to the reaction product after step a), but before cooling the reaction product High levels of acid (e.g., greater than 5%) can add potential risk of corrosion and can impact crystal shape and distribution. Traces of acid in the product can have a negative effect on the thermal stability of the product.

It is preferred that the crystallisation of step b) is performed in the presence of a limited amount of water. A ccordi ngly, the amount of water present during the crystallisation of step b) may be 5 % or less of the reaction product, preferably 4 % or less, even more preferably 3 % or less. The crystallisation of step b) may also be performed in the absence of water.

The crystallisation may be performed in a conventional manner by controlled cooling. Preferably, crystallisation is performed in a single step, but crystallisation may also comprise two or more steps in series.

The reaction product of step a) is typically cooled to a temperature of 40-70 °C so as to crystallise the bisphenol A/phenol adduct to prepare a slurry. Typically, the pure bisphenol A/phenol adduct that forms during the crystallisation can have a molar composition of about 1:1. On a weight basis the composition may typically comprise about 70.8 % by total weight of the bisphenol A/phenol adduct of bisphenol A and about 29.2 % by total weight of the bisphenol A/phenol adduct of phenol. The cooling may, for instance, be carried out by means of an external heat exchanger or by a vacuum cooling crystallisation method in which the reaction product of step a) is cooled down by adding water thereto to make use of vaporisation latent heat of water under reduced pressure. A relatively high crystallisation temperature can result in higher purity, but the crystal yield will be lower. A relatively low crystallisation temperature can provide desirable yield but with lower purity. Preferably, the crystallisation temperature is in the range of 50-60 °C.

Cooling may be performed in multiple subsequent cooling stages in order to achieve even higher purity. For example, a first cooling stage may be followed by a dwell time, after which a second cooling stage may be performed, with an optional subsequent second dwell time and an optional subsequent third cooling stage.

The cooling may represent (or each of the cooling stages may independently represent) a cooling of the mixture with 5-40 °C, such as 10-35 °C or 15-20 °C, using a cooling rate of 0.01-1 °C/min, such as 0.02-0.5 °C /min. Each of the dwell times can last 20-120 minutes, such as 30-90 minutes. A smaller cooling rate can result in a higher purity product, but takes more time. Similarly, a longer dwell time can result in higher purity but takes more time.

Next, the slurry containing the bisphenol A/phenol adduct crystals can be separated into the bisphenol A /phenol adduct and the crystallisation mother liquid containing reaction by-products by conventional means such as filtering and centrifugal separation. A part of the crystallisation mother liquid may be recycled in the reactor or to the crystallisation raw material. To obtain the bisphenol A product without any phenol, e.g. for the production of polycarbonate or epoxy resins, the phenol is removed by first melting the adduct and then stripping the phenol out of the molten mixture. The bisphenol A is then solidified, e.g. in a flaking or prilling unit.

Optionally, the obtained crystals may be subjected to washing, such as with liquid phenol. After filtration, the crystals can be obtained.

Preferably, the obtained bisphenol A has a purity of 99.70 % or more (by total weight of the obtained product on phenol free basis), preferably 99.80 % or more, more preferably 99.90 % or more.

It is preferred that the crystallisation step is carried out in a single step. However, it is also possible that the crystallisation is carried out in two or more steps in seri es.

It is also disclosed the use of acid in a method for crystallising bisphenol A/phenol adduct from a mixture comprising
- at least 10 % by total weight of the mixture of bisphenol A, preferably 15-40 %,
- 60-85 % by total weight of the mixture of phenol,
- 0-5 % by total weight of the mixture of water, preferably 0-3 %,
- 0-2 % by total weight of the mixture of acetone, preferably 0-0.5 %; and/or
- 0-20 % by total weight of impurities, preferably 2-16 %. The weight percent of the mixture will total 100 wt%.

It is further disclosed bisphenol A/phenol adduct crystals obtainable by the method according to the invention, wherein said bisphenol A has a purity of 99.80 % or more (by total weight of the obtained product on phenol free basis). Preferably, the bisphenol A has a purity of 99.90 % or more. Preferably, such bisphenol A/phenol adduct crystals have been obtained with a single crystallisation step.

The use of the terms "a" and "an" and "the" and similar referents in the context of describing the invention (especially in the context of the claims) are to be construed to cover both the singular and the plural, unless otherwise indicated herein or clearly contradicted by context. The terms "comprising", "having", "including" and "containing" are to be construed as open-ended terms (i.e., meaning "including, but not limited to") unless otherwise noted. Recitation of ranges of values herein are merely intended to serve as a shorthand method of referri ng individually to each separate value falling within the range, unless otherwise indicated herein, and each separate value is incorporated into the specification as if it were individually recited herein. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essenti al to the practi ce of the i nventi on. Also, all ranges include any combi nati on of the maximum and minimum points disclosed and include any intermediate ranges therein, which may or may not be specifically enumerated herein. The amounts of components in a product, composition, or mixture, total 100 wt%.

For the purpose of clarity and a concise description features are described herein as part of the same or separate embodiments, however, it will be appreciated that the scope of the i nventi on may include embodiments having combinations of all or some of the features described.

The invention will now be further illustrated by the following non-limiting examples.

### Examples

Experimental work was done using a Lab Max™ system coupled with two probe based instruments: FBRM (Focused Beam Reflectance Measurement) and PV M (Particle Video Microscope) all supplied by Mettler Toledo™. The Lab Max is a sophisticated, fully automated temperature control unit It is capable of controlling lab crystalliser temperature very accurately. It can also follow programmed cooling and heating curves. The lab crystalliser is a jacketed glass vessel equipped with a stirrer. The stirrer speed is also controlled by the Lab Max. The system is equipped with an automated dosing unit. This unit is used to add water or another chemical in different experiments.

FBRM is a laser based instrument. The probe is attached on the top of the crystalliser and reaches into the crystallising solution. Inside the probe is a laser light source. The laser light is sent through a rotational optic lens at the tip of the probe. In this way the laser beam scans through the solution in a circular motion. The linear velocity of the circulation is set to 2 m/s, and it can be adjusted. Once the beam crosses through the solid particle (or any other object, for example a bubble, which has different refractive index) the laser light is back scattered, and received by the sensors in the probe. In this way the probe is able to detect chord lengths of the crystals through which the beam scans.

The PV M is the second probe instrument, and it is also attached on the top of the crystalliser in similar fashion as RFBRM. This probe also reaches into the solution, and it is capable of taking real time photos. The photos are of the dimensions of 800 with 1000 micrometers.

Each of the FBRM, the Lab Max and the PV M are connected to a personal computer.

In order to filter the slurry after the crystallisation is complete a jacketed lab filtration unit from BHS Sonhofen™ is used. This allows for filtration temperatures to be the same as the end of the lab crystallisation protocol. A PE E K filter cloth is used in the filter and a small excess nitrogen pressure (0.05 barg) is applied to filter and wash the cake.

T he feed mixture typically contained 25 % by total weight of the feed mixture of p, p-B PA, 3 % by total weight of the feed mixture of o,p-BPA and 6 % by total weight of the feed mixture of other BPA isomers in phenol and a small amount of water (about 2 % by total weight of the feed mixture). The balance of the mixture comprises of phenol.

### Crystallisation and filtration procedure: protocol 1

Weigh 380 g of the feed mixture in a 1 l glass reactor at 80 °C. Add a known amount of the desired chemical component and set the stirrer at 400 rpm. Stabilise at 80 °C for 30 minutes, cool to 68.5 °C at a rate of 0.1 °C /min. Induce nucleation by gently adding 1 % of finely crushed p,p-BPA crystals (99.95 % purity). Keep the temperature constant for 30 minutes at nucleation temperature. Cool to 60 **°C** with a rate of 0.1 °C /min, keep the temperature at 60 °C during 60 minutes. Drain the slurry in the preheated (60 °C ) filtration unit, leave crystals settle for 5 minutes. Filtrate at 60 °C with a nitrogen pressure of 0.05 barg. Add 40 g of liquid phenol (temperature 75 °C). Filtrate cake completely (no weight increase on scale). Purity is determined by HPLC (High Pressure Liquid Chromatography) analysis on a phenol free basis.

In case strong acids are used a second addition of 50 ml demineralised water can be used (protect the HPLC equipment) without changing the organic composition of the crystals.

### Crystallisation and filtration procedure: protocol 2

Weigh 380 g of the feed mixture in a 1 l glass reactor at 80 °C. Add a known amount of the desired chemical component and set the stirrer at 400 rpm. Stabilise at 80 °C for 30 minutes, cool to 68.5 °C at a rate of 0.1 éC/min. Induce nucleation by gently adding 1 % of finely crushed p,p-BPA crystals (99.95 % purity). Keep the temperature constant for 30 minutes at nucleation temperature. Reduce the temperature with 1 °C (cooling rate 0.025 °C /min) and keep stable for 30 minutes. Cool to 60 °C with a rate of 0.0.25 °C /min, keep the temperature at 60 °C during 60 minutes. Drain the slurry in the preheated (°C éC) filtration unit, leave crystals settle for 5 minutes. Filtrate at 60 °C with a nitrogen pressure of 0.05 barg. Add 40 g of liquid phenol (temperature 75 °C). Filtrate cake completely (no weight increase on scale). Purity is determined by HPLC (High Pressure Liquid Chromatography) analysis on a phenol free basis.

In case strong acids are used a second addition of 50 ml demineralised water can be used (protect the HPLC equipment) without changing the organic composition of the crystals.

### Reference experiment 1

Follows protocol 1 with no addition of any chemical component The total amount of by-products was 5970 parts per million by weight (ppm) of which 2840 ppm o,p-BPA (on phenol free basis). The cake was easily filterable and reached constant weight fast.

### Reference experiment 2

Follows protocol 2 with no addition of any chemical. The total amount of by-products was 1500 ppm of which 840 ppm o,p-BPA (on phenol free basis). The cake was easily filterable and reached constant weight fast

### Comparative Experiments 1-7

These experiments were done following protocol 1 with the addition of a number of different chemicals. This first group of experiments showed little effect or enhanced the co-crystallisation and in general showed poor filterability observed as a poor drained cake. In most cases it was visually observed that the crystals became very fine. The experiments are summarised in Table 1.

**Table 1**

| Comp. Exp. | Component | Amount added (wt.%) | Total amount impurities (wt% phenol free) | Total amount o,p-BPA (wt% phenol free) |
|---|---|---|---|---|
| 1 | cyclohexane | 10 | 0.594 | 0.290 |
| 2 | 1-propanol | 10 | 0.275 | 0.160 |
| 3 | diethylphthalate | 10 | 0.436 | 0.220 |
| 4 | polyethylene glycol 300 | 10 | 0.774 | 0.360 |
| 5 | methylhydrochinon | 10 | 0.853 | 0.400 |
| 6 | benzylalcohol | 10 | 0.344 | 0.191 |
| 7 | mineral oil | 8.7 | 0.267 | 0.148 |

### Experiments 8-14

In this set of experiments following protocol 1, organic acids with various dissociation constants were used. The results are summarised in Table 2. The oxalic acid as strong oxidising agent might have generated other problems.

**Table 2**

| Exp. | Component | Amount added (wt.%) | Total amount impurities (wt% phenol free) | Total amount o,p-BPA (wt% phenol free) |
|---|---|---|---|---|
| 8 | p-hydroxy benzoic acid | 3.0 | 0.244 | 0.146 |
| 9 | 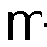 -tartaric acid | 5.0 | 0.112 | 0.080 |
| 10 | 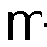 -tartaric acid | 1.0 | 0.331 | 0.166 |
| 11 | 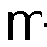 -tartaric acid | 5.0 | 0.387 | 0.197 |
| 12 | oxalic acid | 3.0 | 0.551 | 0.254 |
| 13 | p-tol uene sulphonic acid | 3.4 | 0.151 | 0.108 |
| 14 | p-tol uene sulphonic acid | 4.0 | 0.129 | 0.096 |

### Experiments 15-18

Following protocol 1 using sulphuric acid (96 wt.% in water) and phosphoric acid (85 wt% in water) gave some improvement Since the added sulphuric acid did not dissolve and mai ntai ned as separate liquid phase, the overall effect was not as good as expected and also the filterability was poor. With phosphoric acid addition the resulting cake showed good filterability. The results are summarised in Table 3.

**Table 3**

| Exp. | Component | Amount added (wt.%) | Total amount impurities (wt% phenol free) | Total amount o,p-BPA (wt% phenol free) |
|---|---|---|---|---|
| 15 | H₂SO₄ | 2.8 | 0.331 | 0.166 |
| 16 | H₂SO₄ | 1.4 | 0.379 | 0.178 |
| 17 | H₃PO₄ | 0.1 | 0.273 | 0.144 |
| 18 | H₃PO₄ | 1.0 | 0.317 | 0.159 |

### Experiments 19-29

Following protocol 1 using hydrochloric acid (37wt.% HCI in water) and nitric acid. Both gave significant suppression of the co-crystallisation and thus a high purity product, but also generated a crystal cake that had excellent filterability. The results are summarised in Table 4

**Table 4**

| Exp. | Component | Amount added (wt.%) | Total amount impurities (wt% phenol free) | Total amount o,p-BPA (wt% phenol free) |
|---|---|---|---|---|
| 19 | HCl | 1.07 | 0.108 | 0.076 |
| 20 | HCl | 0.19 | 0.111 | 0.079 |
| 21 | HCl | 0.04 | 0.124 | 0.085 |
| 22 | HCl | 0.56 | 0.101 | 0.070 |
| 23 | HNO₃ | 0.020 ^{(a)} | 0.112 | 0.076 |
| 24 | HNO₃ | 0.014 ^{(a)} | 0.131 | 0.090 |
| 25 | HNO₃ | 0.014 ^{(b)} | 0.133 | 0.081 |
| 26 | HNO₃ | 0.13^{(c)} | 0.100 | 0.066 |
| 27 | HNO₃ | 0.32^{(d)} | 0.102 | 0.067 |
| 28 | HNO₃ | 0.0024 ^{(a)} | 0.173 | 0.102 |
| 29 | HNO₃ | 0.0048 ^{(a)} | 0.115 | 0.080 |

| | | | | |
|---|---|---|---|---|
| ^{(a)} 65 wt.% HNO₃ in water ^{(b)} 6.5 wt% HNO₃ in water ^{(c)} 22 wt.% HNO₃ in water ^{(d)} 32.5 wt.% HNO₃ in water | | | | |

### Experiment 30

Following protocol 2 using nitric acid. The amount of added active component was 0.0048 wt.% (as 65 wt.% HNO₃ in water) and the total amount of impurities was 0.091 wt.% (phenol free) and the amount of o,p-BPA was 0.062 wt.% (on phenol free basis). This relates to the reference experiment 2. Weight percentages refer to the amount of added component in total mixture and o,p-BPA in crystal on phenol free basis.

## Claims

1. A method for the manufacture of bisphenol A, comprising:
a) reacti ng phenol and acetone in the presence of an acidic catalyst to form a reaction product comprising bisphenol A; and
b) crystallising a bisphenol A /phenol adduct from said reaction product, wherein said crystallisation is carried out in the presence of an acid, wherein total amount of acid present during the crystallisation step is 5 % or less by total weight of the reaction product

2. The method of Claim 1, wherein total amount of acid present during the crystallisation step is 4 % or less by total weight of the reaction product, preferably 3 % or less.

3. The method of any of the preceding claims, wherein the amount of water during the reaction of step a) is 5 % or less by total weight of the reaction mixture.

4. The method of any of the preceding claims, wherein the amount of water during the crystallisation of step b) is 5 % or less by total weight of the reaction mixture.

5. The method of any of the preceding claims, wherein said acid comprises inorganic acid, preferably one or more selected from the group consisting of hydrochloric acid, phosphoric acid, and nitric acid.

6. The method of any of the preceding claims, wherein said acid comprises organic acid, preferably organic acid having a pKₐ of at most 3.3.

7. The method of any of the preceding claims, wherein said acid comprises inorganic acid, and wherein the amount of inorganic acid is 1 % or less by total weight of the reaction product, preferably 0.5 % or less, more preferably 0.3 % or less.

8. The method of any of the preceding claims, wherein said acid comprises organic acid, and wherein the amount of organic acid is 0.5-5 % by total weight of the reaction product.

9. The method of any of the preceding claims, wherein the acid is added to the reaction product after step a), but before cooling the reaction product.

10. The method of any of the precedi ng claims, wherein the product mixture comprises:
- at least 10 % by total weight of the reaction product of bisphenol A, preferably 15-40 %;
- 60-85 % by total weight of the reaction product of phenol;
- 0-5 % by total weight of the reaction product of water, preferably 0-3 %;
- 0-8 % by total weight of the reaction product of acetone, preferably 0-5 %; and
- 0-20 % by total weight of the reaction product of by-products, preferably 2-16 %;
wherein the weight totals 100%.

11. The method of any of the preceding claims, wherein the manufactured bisphenol A has a purity of 99.70 % or more on phenol free basis, preferably 99.80 % or more, more preferably 99.90 % or more.

12. The method of any of the preceding claims, wherein the crystallisation is carri ed out in a single step.

13. The method of any of the preceding claims, wherein the crystallisation is carried out in two or more steps in series.

## Patentansprüche

1. Verfahren zum Herstellen von Bisphenol A, umfassend:
a) Umsetzen von Phenol und Aceton in Gegenwart eines sauren Katalysators zum Bilden eines Reaktionsprodukts, das Bisphenol A enthält; und
b) Kristallisieren eines Bisphenol-A/Phenol-Addukts aus dem Reaktionsprodukt, wobei die Kristallisation in Gegenwart einer Säure durchgeführt wird, wobei die Gesamtmenge der Säure, die während des Kristallisationsschritts vorhanden ist, 5 % oder weniger des Gesamtgewichts des Reaktionsprodukts beträgt.

2. Verfahren nach Anspruch 1, wobei die Gesamtmenge der Säure, die während des Kristallisationsschritts vorhanden ist, 4 % oder weniger des Gesamtgewichts des Reaktionsprodukts beträgt, vorzugsweise 3 % oder weniger.

3. Verfahren nach einem der vorhergehenden Ansprüche, wobei die Wassermenge während der Reaktion von Schritt a) 5 % oder weniger des Gesamtgewichts des Reaktionsgemisches beträgt.

4. Verfahren nach einem der vorhergehenden Ansprüche, wobei die Wassermenge während der Kristallisation von Schritt b) 5 % oder weniger des Gesamtgewichts des Reaktionsgemisches beträgt.

5. Verfahren nach einem der vorhergehenden Ansprüche, wobei die Säure vorzugsweise eine oder mehrere anorganische Säuren umfasst, ausgewählt aus der Gruppe bestehend aus Salzsäure, Phosphorsäure und Salpetersäure.

6. Verfahren nach einem der vorhergehenden Ansprüche, wobei die Säure organische Säure umfasst, vorzugsweise eine organische Säure mit einem pKₐ von höchstens 3,3.

7. Verfahren nach einem der vorhergehenden Ansprüche, wobei die Säure anorganische Säure umfasst und wobei die Menge der anorganischen Säure 1 % oder weniger des Gesamtgewichts des Reaktionsprodukts, vorzugsweise 0,5 % oder weniger, stärker bevorzugt 0,3 % oder weniger beträgt.

8. Verfahren nach einem der vorhergehenden Ansprüche, wobei die Säure organische Säure umfasst und wobei die Menge an organischer Säure 0,5 bis 5 % des Gesamtgewichts des Reaktionsprodukts beträgt.

9. Verfahren nach einem der vorhergehenden Ansprüche, wobei die Säure dem Reaktionsprodukt nach dem Schritt a) zugegeben wird, aber bevor das Reaktionsprodukt abkühlt.

10. Verfahren nach einem der vorhergehenden Ansprüche, wobei das Produktgemisch umfasst:
- mindestens 10 % des Gesamtgewichts des Umsetzungsprodukts Bisphenol A, vorzugsweise 15 bis 40 %;
- 60 bis 85 % des Gesamtgewichts des Reaktionsprodukts Phenol;
- 0 bis 5 % des Gesamtgewichts des Reaktionsprodukts Wasser, vorzugsweise 0 bis 3 %;
- 0 bis 8 % des Gesamtgewichts des Reaktionsprodukts Aceton, vorzugsweise 0 bis 5 %; und
- 0 bis 20 % des Gesamtgewichts des Reaktionsprodukts an Nebenprodukten, vorzugsweise 2 bis 16 %;
wobei das Gesamtgewicht 100 % beträgt.

11. Verfahren nach einem der vorhergehenden Ansprüche, wobei das hergestellte Bisphenol A eine Reinheit von 99,70 % oder mehr auf phenolfreier Basis aufweist, bevorzugt 99,80 % oder mehr, stärker bevorzugt 99,90 % oder mehr.

12. Verfahren nach einem der vorhergehenden Ansprüche, wobei die Kristallisation in einem einzigen Schritt durchgeführt wird.

13. Verfahren nach einem der vorhergehenden Ansprüche, wobei die Kristallisation reihenweise in zwei oder mehreren Schritten durchgeführt wird.

## Revendications

1. Méthode de fabrication de bisphénol A, comprenant :
a) la réaction de phénol et d'acétone en présence d'un catalyseur acide pour former un produit réactionnel comprenant du bisphénol A ; et
b) la cristallisation d'un adduit de bisphénol A/phénol à partir dudit produit réactionnel,
dans laquelle ladite cristallisation est mise en œuvre en présence d'un acide, et dans laquelle la quantité totale d'acide présent durant l'étape de cristallisation est de 5 % ou moins du poids total du produit réactionnel.

2. Méthode selon la revendication 1, dans laquelle la quantité totale d'acide présent durant l'étape de cristallisation est de 4 % ou moins du poids total du produit réactionnel, de préférence de 3 % ou moins.

3. Méthode selon l'une quelconque des revendications précédentes, dans laquelle la quantité d'eau durant la réaction de l'étape a) est de 5 % ou moins du poids total du mélange réactionnel.

4. Méthode selon l'une quelconque des revendications précédentes, dans laquelle la quantité d'eau durant la cristallisation de l'étape b) est de 5 % ou moins du poids total du mélange réactionnel.

5. Méthode selon l'une quelconque des revendications précédentes, dans laquelle ledit acide comprend un acide inorganique, de préférence un ou plusieurs choisis dans le groupe constitué par l'acide chlorhydrique, l'acide phosphorique et l'acide nitrique.

6. Méthode selon l'une quelconque des revendications précédentes, dans laquelle ledit acide comprend un acide organique, de préférence un acide organique ayant un pKₐ d'au plus 3,3.

7. Méthode selon l'une quelconque des revendications précédentes, dans laquelle ledit acide comprend un acide inorganique, et dans laquelle la quantité d'acide inorganique est de 1 % ou moins du poids total du produit réactionnel, de préférence de 0,5 % ou moins, mieux encore de 0,3 % ou moins.

8. Méthode selon l'une quelconque des revendications précédentes, dans laquelle ledit acide comprend un acide organique, et dans laquelle la quantité d'acide organique est de 0,5 à 5 % du poids total du produit réactionnel.

9. Méthode selon l'une quelconque des revendications précédentes, dans laquelle l'acide est ajouté au produit réactionnel après l'étape a), mais avant le refroidissement du produit réactionnel.

10. Méthode selon l'une quelconque des revendications précédentes, dans laquelle le mélange de produits comprend :
- au moins 10 %, du poids total du produit réactionnel, de bisphénol A, de préférence 15 à 40 % ;
- 60 à 85 %, du poids total du produit réactionnel de phénol ;
- 0 à 5 %, du poids total du produit réactionnel d'eau, de préférence 0 à 3 % ;
- 0 à 8 %, du poids total du produit réactionnel d'acétone, de préférence 0 à 5 % ; et
- 0 à 20 %, du poids total du produit réactionnel de sous-produits, de préférence 2 à 16 % ;
dans laquelle le poids est au total de 100 %.

11. Méthode selon l'une quelconque des revendications précédentes, dans laquelle le bisphénol A manufacturé a une pureté de 99,70 % ou plus sur une base sans phénol, de préférence de 99,80 % ou plus, mieux encore de 99,90 % ou plus.

12. Méthode selon l'une quelconque des revendications précédentes, dans laquelle la cristallisation est effectuée en une seule étape.

13. Méthode selon l'une quelconque des revendications précédentes, dans laquelle la cristallisation est effectuée en deux étapes ou plus en série.
